Europäisches Patentamt

(19) European Patent Office

Office européen des brevets

(11) Publication number: **0 119 020**
**B1**

(12) **EUROPEAN PATENT SPECIFICATION**

(45) Date of publication of patent specification: **01.06.88**

(21) Application number: **84300949.9**

(22) Date of filing: **14.02.84**

(51) Int. Cl.⁴: **A 61 K 9/50**

(54) **Method of preparing liposomes and products produced thereby.**

(30) Priority: **15.02.83 GB 8304165**
**03.10.83 GB 8326448**
**06.01.84 GB 8400306**

(43) Date of publication of application:
**19.09.84 Bulletin 84/38**

(45) Publication of the grant of the patent:
**01.06.88 Bulletin 88/22**

(84) Designated Contracting States:
**AT BE CH DE FR GB IT LI LU NL SE**

(56) References cited:
**EP-A-0 087 993**
**GB-A-2 002 319**
**GB-A-2 013 609**

**CHEMICAL ABSTRACTS, vol. 88, no. 6, 6th
February 1978, page 253, no. 41686p,
Columbus, Ohio, USA**

(73) Proprietor: **E.R. Squibb & Sons, Inc.**
**Lawrenceville-Princeton Road**
**Princeton, N.J. 08540 (US)**

(72) Inventor: **Ridgway, Frank**
**"Bellefield" Noctorum Lane**
**Noctorum Birkenhead Merseyside (GB)**
Inventor: **Payne, Nicholas I.**
**9 Antons Road Pensby**
**Wirral Merseyside (GB)**
Inventor: **Timmins, Peter**
**34 Thornley Road Moreton**
**Wirral Merseyside (GB)**
Inventor: **Groom, Cheryl Vanessa**
**1 West Cottages Leadpipe Lane**
**Cotherstone Barnard Castle County Durham**
**(GB)**

(74) Representative: **Thomas, Roger Tamlyn et al**
**D. Young & Co. 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Field of the invention

The present invention relates to a method for preparing liposome precursors in the form of particulate carrier materials coated with thin films of liposome components, which coated carrier materials are employed to form liposome preparations, and to intermediates and products produced in such method.

### Background of the invention

Liposomes are widely described in the literature and their structure is well known. They are formed by amphipathic molecules such as the class II polar lipids, that is, phosphatidyl cholines, ethanolamines and serines, sphingomyelins, cardiolipins, plasmalogens, phosphatidic acids and cerebrosides. Liposomes are formed when phospholipids or other suitable amphipathic molecules are allowed to swell in water or aqueous solution to form liquid crystals usually of multilayer structure comprised of many bilayers separated from each other by aqueous material. Another type of liposome is known consisting of a single bilayer encapsulating aqueous material which may also be referred to as a unilamellar vesicle. "If water-soluble materials are included in the aqueous phase during the swelling of the lipids they become entrapped between the lipid bilayers. Alternatively, lipid soluble materials may be dissolved in the lipid and, hence, may be incorporated into the lipid bilayers themselves," Ryman, B. E., "The Use of Liposomes as Carriers of Drugs and other Cell-Modifying Molecules," Proc. 6th Int'l. Congr. Pharmacol. 5,91 (1976), published in "Drug Applications," *Clinical Pharmacology*, Vol. 5, pp.91—103, Pergamon Press (1975).

In recent years there has been much interest in the use of liposomes as carriers of compounds which are of interest Because of one or other biological property, for example, medicaments, proteins, enzymes, hormones and diagnostic agents, hereinafter referred to as "biologically active compounds". Liposomes have been suggested as carriers for drugs, see Ryman, supra at page 91 and Gregoriadis, G., "Enzyme or Drug Entrapment in Liposomes: Possible Biomedical Application," *Insolubilized Enzymes,* Ed. M. Salmona et al, Raven Press, N.T. 1974, pp. 165—177.

Water-soluble materials are encapsulated in the aqueous spaces between the biomolecular layers. Lipid soluble materials are incorporated into the lipid layers although polar head groups may protrude from the layer into the aqueous space. The encapsulation of these compounds can be achieved by a number of methods. The method most commonly used involves casting a thin film of phospholipid onto the walls of a flask by evaporation of an organic sovent. When this film is dispersed in a suitable aqueous medium, multilamellar liposomes are formed (also referred to as coarse liposomes). Upon suitable sonication, the coarse liposomes form smaller similarly closed vesicles.

Water-soluble biologically active compounds are usually incorporated by dispersing the cast film with an aqueous solution of the compound. The unencapsulated compound is then removed by centrifugation, chromatography, dialysation or some other suitable procedure. Lipid-soluble biologically active compounds are usually incorporated by dissolving them in the organic solvent with the phospholipid prior to casting the film. Providing the solubility of these compounds in the lipid phase is not exceeded or the amount present is not in excess of that which can be bound to the lipid, liposomes prepared by the above method usually contain most of the compound bound in the lipid bilayers; separation of the liposomes from unencapsulated material is not required. Other methods of preparing liposomes have been described although these are mainly specialized methods producing unilamellar liposomes and include reverse-phase evaporation of an organic solvent from a water-in-oil emulsion of phospholipid, infusion of organic solutions of phospholipid into large volumes of aqueous phase and detergent removal from mixed micelles of detergent and lipid.

Aqueous liposome dispersions only have limited physical stability. The liposomes can aggregate and precipitate as sediment. Although this sediment may be redispersed, the size distribution may be different from that of the original dispersion. This may be overcome to some extent by incorporation of charged lipids into the liposomes. In addition, on storage the biologically active compounds may be lost into the external aqueous phase which restricts the potential of these preparations as practical dosage forms. This is particularly notable for low molecular weight water-soluble compounds but lipid soluble compounds too can partition into the external aqueous medium. If the volume of the aqueous medium is large, this loss can be significant. In addition, depending upon the type of lipid and biologically active compound present in the liposome, there is the potential for chemical degradation of the lipid components and/or the biologically active components in the aqueous dispersion.

These factors restrict the use of liposomes as practical carriers of biologically active compounds. One solution suggested for overcoming the limited physical stability of liposomes is to prepare and store the lipid/biologically active compound film and then disperse the film to form liposomes just prior to administration. However, unit dose film preparation presents serious practical difficulties in that the containers would require a high surface area to facilitate solvent evaporation and deposition of a thin film suitable for rapid rehydration to form liposomes readily. This type of container by virtue of its bulk would present severe storage problems. Other methods suggested for preparing liposome components in a solid form for storage have included freeze-drying the prepared aqueous liposome suspension as described in U.S. Patents. (US—A—) 4 229 360 and (US—A—) 4 247 411 and by freeze-drying the liposome components from a suitable orgainic solvent as describe in U.S. Patent (US—A—) 4 311 712. These freeze-dried preparations result in a porous matrix of liposome components which is easily hydrated.

Chem. Abstr. 88 (1978), Abstr. No. 41686p describes a method for preparing a liposome precursor comprising dissolving egg lecithin in $CHCl_3$ and coating it on glass beads.

Brief description of the invention

In accordance with the present invention, a method is provided for preparing a stable liposome precursor in the form of a thin film of liposomal components coated on a particulate carrier material, which comprises forming a solution in a suitable solvent of at least one liposome forming lipid, optionally, at least one biologically active compound, and optionally, at least one adjuvant which imparts advantageous properties to the final liposome preparation, and coating a granular free-flowing carrier material which is substantially insoluble in said solvent, with the so-formed solution to form a thin film of liposomal components on said carrier material, wherein the carrier material is a physiologically acceptable carrier material which has a relatively high water solubility. In a modification of this method, the coating solution is formed by dissolving the optional biologically active compound and optional adjuvant in said lipid, said lipid being of the low melting point liposome-forming type.

In this way thin films of liposome components may be prepared which are not subject to the physical stability problems set out above, and which may be employed to form liposome preparations immediately prior to administration.

In addition, in accordance with the present invention, a method is provided for forming a liposome preparation which method includes the step of exposing the particulate material coated with the thin film of liposome components to water (as such or as a solution containing water) thereby causing the thin film of liposome components to hydrate to give a liposome preparation. As the carrier material is water-soluble, the material dissolves to give a preparation similar to that prepared by hydration of cast films with a solution of the carrier material.

Further, in accordance with the present invention, there is provided the intermediate formed above which is comprised of the relatively stable particulate carrier material coated with a thin film of liposome components and which is useful for forming the liposome preparation.

The problems associated with the physical stability of liposome dispersions on storage may be overcone by forming the aqueous dispersion of the coated powdered carrier material prior to administration. Additionally, the chemical integrity of the biologically active compounds and lipid components may be protected in the coated powdered preparations by the incorporation of antioxidants therein or packing the coated powdered material under inert atmospheres, for example.

Detailed description of the preferred embodiments

The carrier material to be coated may be any physiologically acceptable water-soluble free-flowing powder which, even after processing, will remain substantially granular and free-flowing.

The water-soluble carrier materials should have a high water-solubility, for example, in excess of about 10% by weight in water and a rapid dissolution rate in water, for example, complete solution in 3 to 4 minutes at 40°C, and should be suitable for intravenous use. In addition, such a carrier material should be substantially insoluble in the solvent used for dissolving the mixture of lipid, optional active compound and optional adjuvant and should form an isotonic solution in water in a concentration range of from about 1 to about 10% w/v, and preferably from about 3 to about 7% w/v. Examples of suitable carrier materials include sorbitol, mannitol, xylitol, or naturally occurring amino acids such as arginine or glycine, with sorbitol being preferred. Other water-soluble materials suitable for intravenous use include sodium chloride, lactose, dextrose and sucrose.

It may be advantageous to use micronized forms of the water-soluble carrier materials (that is, having an average particle size of less than about 10 microns) as the high surface area would facilitate the hydration and dissolution of the liposomal components. However, the carrier materials may have an average particle size of up to 500 microns and still be useful. The amount of carrier material used may be adjusted so that the final reconstituted suspension is iso-osmotic with the blood, although for small volume injections this may not be necessary. As a suitable aqueous medium for dispersion, distilled water, isotonic saline or buffer solution may be used, the temperature of which may be modified to exceed the phase transition temperature of the lipid components used in formulation.

In carrying out the method of the invention for preparing the particulate carrier materials coated with a thin film of liposomal components, at least one liposome forming amphipathic lipid, optionally, at least one biologically active compound, and, optionally, at least one adjuvant are dissolved in a solvent and this solution is used to coat a suitable carrier material. For low melting point liposome-forming amphipathic lipids (that is have a melting point below 50°C), the optional biologically active compound and optional adjuvant may be directly dissolved in the lipid and this solution used to coat the carrier material.

The lipid will generally be present in the solution (containing a separate solvent), to be used to coat the carrier material, in an amount of within the range of from about 1 to about 25% by weight, depending upon the solubility of the lipid in the solvent or solvent mixture used, and preferably from about 2.5 to about 12.5% by weight of such solution. The optional biologically active compound and optional adjuvant material will be present in the coating solution in varying amounts depending upon the nature of the particular compound and/or material employed.

The ratio of lipid to optional biologically active compound in the coating solution will depend upon the lipid solubility or binding of the biologically active compound used. Thus, the coating to be applied to the carrier material will normally contain a weight ratio of lipid:optional biologically active compound of within the range of from about 5:1 to about 1000:1 and preferably from about 10:1 to about 200:1 depending upon the particular biologically active compound to be employed. For example, where the biologically active compound is an anti-infective, such as an antibiotic or an anti-fungal agent, the lipid will be present in a weight ratio to the biologically active compound of within the range of from about 5:1 to about 1000:1 and preferably from about 10:1 to about 300:1. Where the biologically active compound is a contrast agent, the lipid will be generally present in a weight ratio to the contrast agent in an amount of within the range of from about 5:1 to about 1000:1 and preferably from about 10:1 to about 200:1.

The amounts of optional adjuvant material and biologically active material employed in the coating will comprise amounts conventionally employed in forming liposomes.

The amount of coating applied to the carrier material will depend upon physical characteristics of the carrier material such as surface area and, the isotonicity requirements. Thus, the coating will normally be present in a weight ratio to carrier material in an amount of within the range of from about 0.03:1 to about 0.3:1 and preferably from about 0.05:1 to about 0.2:1.

Various amphipathic lipid which are known to be suitable for preparing liposomes by known methods can be used in the method of this invention. Thus, a wide variety of lipids may be used but non-immunogenic and biodegradable lipids would be preferred. Examples of suitable lipids are the phospholipids, for example, natural lecithins, such as egg lecithin or soya bean lecithin, or synthetic lecithins such as saturated synthetic lecithins, for example, dimristolyl phosphatidyl choline, dipalmitoyl phosphatidyl choline or distearoyl phosphatidyl choline or unsaturated synthetic lecithins, such as dioleyl phosphatidyl choline oe dilinoleylphosphatidyl choline, with egg lecithin or soya bean lecithin being preferred.

The biologically active compound employed in the present invention may be various compounds of biological interest: for example, the compound may be a medicament, such as an anti-infective, for example amphotericin B, ketoconazole, isoconazole, miconazole and benzyl penicillin, anti-tumor agents, such as 5-fluorouracil, methotrexate, actinomycin D, enzyme, hormone, contrast agent, marker compound or NMR imaging agent, such as 4 - succinyl - 4 - amino - 2,2,6,6 - tetramethylpiperidine - 1 - oxyl.

Examples of contrast agents suitable for use in the present invention include, but are not limited to the following: N,N' - bis[2 - hydroxy - 1 - (hydroxymethyl)ethyl] - 5 - [(2 - hydroxy - 1 - oxopropyl) - amino] - 2,4,6 - triiodo - 1,3 - benzenedicarboxamide (Bracco 15,000), metrizamide, diatrizoic acid, sodium diatrizoate, meglumine diatrizoate, acetrizoic acid and its soluble cationic salts, diprotrizoic acid and its soluble inorganic and organic cationic salts, iodamide, sodium iodipamide, meglumine iodipamide, iodohippuric acid and its soluble salts, iodomethamic acid and its soluble salts, iodopyracetiodo - 2 - pyridone - N - acetic acid and its soluble salts, 3,5 - diiodo - 4 - pyridone - N - acetic acid (iodopyracet), 3,5 - diiodo - 4 - pyridone - N - acetic acid diethanolamine salt, iodo - 2 - pyridone - N - acetic acid and its amine salt, iothalamic acid and its soluble salts, methanesulfonic acid, metrizoic acid and its soluble salts, sodium ipodate, ethiodized oil, iopanoic acid, iocetamic acid, tyropanoate sodium, iopydol, iophenoxic acid, iophendylate, and other chemically related iodinated contrast agents. Unless indicated otherwise, where applicable, the contrast agents which may be employed herein include inorganic, organic and cationic salts of the above contrast agent, such as the potassium salt, calcium salt, lithium salt, arginin salt, cystein salt, glycin salt, glycyl glycin salt, N-methyl glucosamine salt and other non-toxic aliphatic and alicyclic amines employed in preparing water soluble salts. Other X-ray contrast agents which may be employed herein are disclosed in German Offenlegungsschrift (DE—A—) 2 935 195.

The final liposome preparation containing a contrast agent prepared by the method of the invention may be employed as described in U.S. Patent (U.S—A—) 4 192 859.

Other proteins and drugs available for use herein as optional biologically active compounds include steroids such as hydrocortisone, colchicine, insulin, cyclic AMP and α-thiodeoxyguanosine, chelating agents and cell modifying substances, such as antigens and interferon inducers.

The present invention is particularly useful in the case of lipid-soluble or lipid-bound biologically active compounds (which include some water-soluble compounds, such as proteins).

The method of this invention, like other methods of preparing liposomes, will result in partial incorporation of water-soluble biologically active compounds. Usually the formation of liposomes containing this type of compound is followed by removal of the unencapsulated material: however, in some instances coadministration of unencapsulated and liposomally entrapped biologically-active compounds may be advantageous.

The optional adjuvants suitable for use in the present invention may be:

a) substances which are known to provide a negative charge on the liposomes, for example, egg phosphatidic acid or dicetyl phophate;

b) substances known to provide a positive charge, for example, stearyl amine, or stearyl amine acetate;

C) substances shown to affect the physical properties of the liposomes in a more desirable way; for example, caprolactam and/or sterols such as cholesterol, ergosterol, phytosterol, sitosterol, sitosterol pyroglutamate, 7-dehydrocholesterol or lanosterol, may affect membrane rigidity;

D) substances known to have antioxidant properties to improve the chemical stability of the particulate carrier coated with liposome components, such as tocopherol, propyl gallate, ascorbyl palmitate, or butylated hydroxy toluene.

Suitable solvents for use in dissolving or aiding in dissolution of the above-mentioned mixture of lipid and optional biologically active compound and optional adjurant include, but are not limited to, ethanol, methanol, chloroform, dichloromethane, diethyl ether, carbon tetrachloride, ethyl acetate, dioxane, cyclohexane and the like, with methanol, ethanol or chloroform being preferred.

The liposomal components (excluding the biologically active compound) preferably are binary mixtures of lecithin and a sterol selected from the group listed hereinabove, or ternary mixtures of lecithin, dicetyl phosphate, and a sterol selected from the group listed hereinabove, in the preferred molar ratios of 7:2:1, respectively. The molar percentage of lecithin may range from about 55 to about 95% and the sterol from about 5 to about 35% based on a binary mixture. The molar percentage of lecithin may range from about 50% to about 80%, the dicetyl phosphate from 0 to about 30%, and the sterol from about 5 to about 30%, based on a ternary lipid mixture. Lecithin is employed to take advantage of its property of swelling in salt solutions. Dicetyl phosphate has the property of imparting the negative charge to the lipid membranes so that the mutual repulsive action of opposing channel surfaces widens the channels.

The components which constitute the liposomal mixture are commerically available or may readily be prepared.

Coating of the carrier material may be achieved by applying solutions of liposomal components dissolved either in the lipid phase or in a suitable organic solvent followed by solvent removal. Alternatively, the carrier material may be spray dried in the presence of a solution of liposomal components. By suitable containment and sterilization of component materials, sterile carrier material coated with the liposomal components is produced. The coated carrier material of the invention may be packed in sterile unit dose vials under aseptic conditions and reconstituted immediately prior to use by the physician, for example, by adding water, saline or buffer solution (with vigorous shaking and centrifuging or filtering to remove insoluble carrier material).

The final liposome formulations prepared as described above may be administered parenterally, for example, intravenously, as well as orally and topically.

The following Examples represent preferred embodiment of the present invention. All temperatures unless otherwise indicated are expressed in degrees Centigrade.

Example 1

Five hundred and ten milligrams (510 mg) of dextrose (anhydrous) were placed in a 100 ml round bottom flask and 40 mg of egg lecithin dissolved in 3 ml of chloroform added in 3×1 ml portions. After each portion addition, the solvent was removed under vacuum from the rotating flask. Microscopic examination of the powder showed that the dextrose particles were coated with lipid material.

The so-formed coated carrier may be packaged and stored in sterile unit dose vials under aseptic conditions and reconstituted immediately prior to use.

Distilled water (5 ml) was added to a portion of the above dextrose preparation coated with phospholipid (225 mg) in a vial and the mixture heated to 60°C for one minute, then agitated by hand to achieve the final dispersion. The size distribution of the liposome preparation was log-normal as determined using a Coulter Counter with a mass median volume equivalent diameter of 5.3μm and geometric standard deviation of 1.58.

The above liposome preparation is similar to those prepared by hydration of cast films with a solution of the carrier material.

Example 2

Egg lecithin (2.0 g), ergosterol (0.5g) and amphotericin B (50.0 mg) were dissolved in methanol (10ml). Lactose (13.0g) was placed in a 250 ml round bottom flask and the above solution added in 2 ml portions. After each portion addition, the solvent was removed under vacuum from the rotating flask. Microscopic examination of the powder showed that the lactose particles were coated with lipid material.

The so-formed coated carrier may be packaged and stored in sterile unit dose vials under aseptic conditions and reconstituted immediately prior to use by the physician as follows.

Water for injection (10 ml) was added to a portion of the above lactose preparation coated with liposomal components (0.775 g) in a vial and the mixture heated to about 60—70°C in a water bath to aid dissolution of the carrier material. Shaking of the vial caused the preparation to disperse resulting in a milky dispersion. The size distribution of the liposomes formed was log-normal as determined using a Coulter Counter with a mass mediam volume equivalent diameter of 2.5μm and a geometric standard deviation of 1.56.

The liposome preparation containing the amphotericin B is similar to those prepared by hydration of cast films with a solution of the carrier material.

Example 3

Egg lecithin (89 mg), cholesterol (24 mg), dicetyl phosphate (12.4 mg) and amphotericin B (2.6 mg) were dissolved in methanol (10 ml). Sorbitol (500 mg) was placed in a 250 ml round bottom flask and the above solution added in 2 ml portions. After each portion addition, the solvent was removed under vacuum from the rotating flask. Microscopic examination of the powder showed that the sorbitol particles were coated with lipid material.

The so-formed coated carrier may be packaged and stored in sterile unit dose vials under aseptic conditions and reconstituted immediately prior to use by the physician as follows.

Water for injection (10 ml) was added to a portion of the above sorbitol preparation coated with liposomal components (0.628 g) in a vial and the mixture heated at 60—70°C to dissolve the carrier material and form a liposome preparation.

The liposome preparation containing the amphotericin B is similar to those prepared by hydration of cast films with a solution of the carrier material.

Example 4

Egg lecithin (121 mg), ergosterol (5 mg) and amphotericin B (5 mg) were dissolved in methanol (10 ml). Sorbitol (500 mg) was placed in 250 ml round bottom flask and the above solution added in 2 ml portions. After each portion addition, the solvent was removed under vacuum from the rotating flask. Microscopic examination of the powder showed that the sorbitol particles were coated with lipid material.

The so-formed coated carrier may be packaged and stored in sterile unit dose vials under aseptic conditions and reconstituted immediately prior to use by the physician as follows.

Water for injection (10 ml) was added to a portion of the above sorbitol preparation coated with liposomal components (0.631 g) in a vial and the mixture heated at 60—70°C to dissolve the carrier material and form a liposome preparation.

The liposome preparation containing the amphotericin B is similar to those prepared by hydration of cast films with a solution of the carrier material.

**Claims**

1. A method for preparing a stable liposome precursor in the form of a thin film of liposomal components coated on a particulate carrier material, which comprises forming a solution in a suitable solvent of at least one liposome-forming lipid, optionally, at least one biologically active compound, and optionally, at least one adjuvant which imparts advantageous properties to the final liposome preparation, and coating a granular free-flowing carrier material which is substantially insoluble in said solvent, with the so-formed solution to form a thin film of liposomal components on said carrier material, wherein the carrier material is a physiologically acceptable carrier material which has a relatively high water solubility.

2. The method as defined in claim 1 wherein said carrier material has a water-solubility in excess of 10% by weight, a rapid dissolution rate in water, and will form an isotonic solution in water in a concentration of from about 1 to about 10% w/v.

3. The method as defined in Claim 1 or 2 wherein said carrier material is a water-soluble carrier suitable for intravenous use.

4. The method as defined in Claim 3 wherein said carrier material is sorbitol, mannitol, or xylitol or a naturally occurring amino acid.

5. The method as defined in Claim 4 wherein said carrier material is sorbitol.

6. The method as defined in Claim 1 wherein the carrier material is sodium chloride, lactose dextrose or sucrose.

7. The method as defined in any preceding claim wherein the solution is formed by dissolving said lipid, optionally, said biologically active compound and, optionally, said adjuvant in one or more organic solvents.

8. The method as defined in Claim 7 wherein the organic solvent is ethanol, methanol, chloroform, dichloromethane, diethylether, carbon tetrachloride, ethyl acetate, dioxane or cyclochexane.

9. The method as defined in Claim 8 wherein the solvent is methanol, ethanol or chloroform.

10. A modification of the method as defined in any one of Claims 1—6 wherein the coating solution is formed by dissolving the optional biologically active compound and optional adjuvant in said lipid, said lipid being of the low melting point liposome-forming type.

11. The method as defined in any one of Claims 1—9 wherein the particulate carrier material is coated with said solution by suspending the carrier material in the solution of liposomal components and spray drying the coated carrier material.

12. The method as defined in any preceding claim wherein the lipid is a phospholipid.

13. The method as defined in Claim 12 wherein the phospholipid is a natural or synthetic lecithin.

14. The method as defined in any preceding claim wherein said solution includes a biologically active compound which is a medicament, contrast agent, enzyme, hormone, marker compound or NMR imaging agent.

15. The method as defined in any preceding claim wherein the adjuvant is egg phosphatidic acid, dicetyl phosphate, or stearyl amine.

16. The method as defined in any one of Claims 1—14 wherein the adjuvant is a sterol.

17. The method as defined in Claim 15 wherein the adjuvant also comprises caprolactam and/or a sterol selected from cholesterol, phytosterol, ergosterol, sitosterol, sitosterol pyroglutamate, 7-dehydrocholesterol, lanosterol and mixtures thereof.

18. The method as defined in any preceding claim wherein the biologically active compound is amphotericin B.

19. The method as defined in Claim 1 wherein the biologically active compound is amphotericin B, said carrier material is sorbitol and said adjuvant includes a sterol which is ergosterol.

20. The method as defined in Claim 1 wherein the lipid is egg lecithin, the adjuvant is ergosterol, cholesterol or dicetyl phosphate, and the carrier material is sorbitol.

21. The method as defined in Claim 1 wherein the lipid is egg lecithin, the adjuvant is ergosterol or cholesterol, and the carrier material is lactose.

22. A stable liposome precursor which when mixed with water forms a liposomal preparation, comprising a particulate carrier material, said carrier material being coated with a thin film comprised of at least one liposome-forming lipid, optionally at least one biologically active compound and, optionally, at least one adjuvant which imparts advantageous properties to the final liposome preparation wherein the carrier material is a water-soluble particulate material.

23. The stable liposome precursor as defined in Claim 22 wherein the carrier material is a free-flowing physiologically acceptable carrier material which will form an isotonic solution in water in a concentration of from about 1 to about 10% w/v.

24. The stable liposome precursor as defined in Claim 23 wherein the carrier material is sorbitol.

25. The stable liposome precursor as defined in any one of Claims 22—24 wherein the biologically active compound is amphotericin B.

26. A stable liposome precursor which when mixed with water forms a liposomal preparation, comprising a water-soluble carrier material coated with a thin film comprised of at least one liposome-forming lipid, optionally, at least one biologically active compound and, optionally, at least one adjuvant which imparts advantageous properties to the final liposome preparation, prepared by the method as defined in any one of Claims 1—21.

27. A method for preparing a liposome preparation which comprises exposing the liposome precursor as defined in any one of Claims 22—26 to water.

**Patentansprüche**

1. Verfahren zur Herstellung eines stabilen Liposomen-Vorläufers in Form eines dünnen Films liposomaler Bestandteile, die auf einem teilchenförmigen Trägermaterial aufgetragen sind, dadurch gekennzeichnet, daß man eine Lösung in einem geeigneten Lösungsmittel von mindestens einem Liposomen-bildenden Lipid, gegebenen falls mindestens einer biologische aktiven Verbindung und gegebenenfalls mindestens einem Adjuvans bildet, das dem fertigen Liposomen-Präparat vorteilhafte Eigenschaften verleiht und ein körniges, freifließendes Trägermaterial, das in dem Lösungsmittel praktisch unlöslich ist, mit der erhaltenen Lösung beschichtet, unter Bildung eines dünnen Films liposomaler Bestandteile auf dem Trägermaterial, und wobei das Trägermaterial ein physiologisch verträgliches Trägermaterial ist, das eine Verhältnismäßig hohe Wasserlöslichkeit aufweist.

2. Verfahren nach Anspruch 1, dadurch genennzeichnet, daß das Trägermaterial eine Wasserlöslichkeit von mindestens 10 Gewichtsprozent besitzt, eine rasche Auflösungsgeschwindigkeit in Wasser hat und in Wasser bei einer Konzentration von etwa 1 bis etwa 10 Gewichtsprozent pro Volumen eine isotonische Lösung bildet.

3. Verfahren nach Anspruch 1 oder 2, dadurch gekennzeichnet, daß das Trägermaterial ein wasserlöslicher Träger ist, der sich zum intravenösen Gebrauch eignet.

4. Verfahren nach Anspruch 3, dadurch gekennzeichnet, daß das Trägermaterial Sorbit, Mannit oder Xylit oder eine natürlich vorkommende Aminosäure ist.

5. Verfahren nach Anspruch 4, dadurch gekennzeichnet, daß das Trägermaterial Sorbit ist.

6. Verfahren nach Anspruch 1, dadurch gekennzeichnet, daß das Trägermaterial Natriumchlorid, Lactose, Dextrose oder Saccharose ist.

7. Verfahren nach einem der vorhergehenden Ansprüche, dadurch gekennzeichnet, daß die Lösung durch Auflösen des Lipids, gegebenenfalls der biologisch aktiven Verbindung und gegebenenfalls des Adjuvans in einem oder mehreren organischen Lösungsmitteln hergestellt wird.

8. Verfahren nach Anspruch 7, dadurch gekennzeichnet, daß das organische Lösungsmittel Äthanol, Methanol, Chloroform, Dichlormethan, Diäthyläther, Tetrachlorkohlenstoff, Äthylacetate, Dioxan oder Cyclohexan ist.

9. Verfahren nach Anspruch 8, dadurch gekennzeichnet, daß das Lösungsmittel Methanol, Äthanol oder Chloroform ist.

10. Abänderung des Verfahrens nach einem der Ansprüche 1 bis 6, dadurch gekennzeichnet, daß die Beschichtungslösung hergestellt wird durch Auflösen der gegebenenfalls verwendeten, biologisch aktiven Verbindung und des gegebenenfalls verwendeten Adjuvans in dem Lipid, wobei das Lipid eine Verbindung ist, die Liposomen mit niedrigem Schmelzpunkt bildet.

11. Verfahren nach einem der Ansprüche 1 bis 9, wobei das teilchenförmige Trägermaterial mit der Lösung durch Suspendieren des Trägermaterials in der Lösung der liposomalen Bestandteile und Sprühtrocknen des beschichteten Trägermaterials beschichtet wird.

12. Verfahren nach einem der vorhergehenden Ansprüche, wobei das Lipid ein Phospholipid ist.

13. Verfahren nach Anspruch 12, wobei das Phospholipid ein natürliches oder synthetisches Lecithin ist.

14. Verfahren nach einem der Vorhergehenden Ansprüche, wobei die Lösung eine biologisch aktive Verbindung enthält, die ein Arzneistoff, ein Kontrastmittel, ein Enzym, ein Hormon, einen Marker-Verbindung oder eine NMR-abbildende Verbindung ist.

15. Verfahren nach einem der vorhergehenden Ansprüche, wobei das Adjuvans eine Eiphosphatidinsäure, Dicetylphosphat oder Stearylamin ist.

16. Verfahren nach einem der Ansprüche 1 bis 14, wobei das Adjuvans ein Sterin ist.

17. Verfahren nach Anspruch 15, wobei das Adjuvans zusätzlich Caprolactam und/oder Cholesterin, Phytosterin, Ergosterin, Sitosterin, Sitosterin -Pyroglutamat, 7-Dehydrocholesterin, Lanosterin oder deren Gemische enthält.

18. Verfahren nach einem der vorhergehenden Ansprüche, wobei die biologisch aktive Verbindung Amphotericin B ist.

19. Verfahren nach Anspruch 1, wobei die biologisch aktive Verbindung Amphotericin B und das Trägermaterial Sorbit ist und das Adjuvans Ergosterin enthält.

20. Verfahren nach Anspruch 1, wobei das Lipid Eilecithin, das Adjuvans Ergosterin, Cholesterin oder Dicetylphosphat und das Trägermaterial Sorbit ist.

21. Verfahren nach Anspruch 1, wobei das Lipid Eilecithin, das Adjuvans Ergosterin oder Cholesterin und das Trägermaterial Lactose ist.

22. Ein stabiler Liposomen-Vorläufer, der nach dem Vermischen mit Wasser ein liposomales Präparat bildet, umfassend ein teilchenförmiges Trägermaterial, wobei das Trägermaterial mit einem dünnen Film aus mindestens einem Liposomen-bildenden Lipid, gegebenenfalls mindestens einer biologisch aktiven Verbindung und gegebenenfalls mindestens einem Adjuvans beschichtet ist, wobei das Adjuvans dem fertigen Liposomen-Präparat vorteilhafte Eigenschaften verleiht, und wobei das Trägermaterial ein wasserlösliches teilchenförmiges Material ist.

23. Stabiler Liposomen-Vorläufer nach Anspruch 22, wobei das Trägermaterial ein freifließendes, physiologisch verträgliches Trägermaterial ist, das in Wasser in einer Konzentration von etwa 1 bis etwa 10 Gewichtsprozent pro Volumen eine isotonische Lösung bildet.

24. Stabiler Liposomen-Vorläufer nach Anspruch 23, wobei das Trägermaterial Sorbit ist.

25. Stabiler Liposomen-Vorläufer nach einem der Ansprüche 22 bis 24, wobei die biologisch aktive Verbindung Amphotericin B ist.

26. Stabiler Liposomen-Vorläufer, der beim Vermischen mit Wasser ein liposomales Präparat bildet, enthaltend ein wasserlösliches Trägermaterial, das mit einem dünnen Film aus mindestens einem Liposomen bildenden Lipid, gegebenenfalls mindestens einer biologisch aktiven Verbindung und gegebenenfalls mindestens einem Adjuvans beschichtet ist, wobei das Adjuvans dem fertigen Liposomen-Präparat vorteilhafte Eigenschaften verleiht, hergestellt nach dem Verfahren gemäß einem der Ansprüche 1 bis 21.

27. Verfahren zu Herstellung eines Liposomen Präparats, dadurch gekennzeichnet, daß man den Liposomen-Vorläufer nach einem der Ansprüche 22 bis 26 mit Wasser zusammenbringt.

## Revendications

1. Procédé de préparation d'un précurseur de liposomes stable sous la forme d'une fine pellicule de composants liposomiques appliquée sur un matériau porteur en particules, procédé qui consiste à former une solution, dans un solvant convenable, d'au moins un lipide formateur de liposomes, facultativement d'au moins un composé biologiquement actif, et facultativement d'au moins un adjuvant qui confère des propriétés avantageuses à la préparation de liposomes finale, et à revêtir un matériau porteur fluide granulaire, qui est sensiblement insoluble dans ledit solvant, de la solution ainsi formée de façon à former une mince pellicule de composants liposomiques sur ledit matériau porteur, procédé dans lequel le matériau porteur est un matériau porteur physiologiquement acceptable qui possède une solubilité dans l'eau relativement grande.

2. Procédé selon la revendication 1, dans lequel ledit matériau porteur a une solubilité dans l'eau de plus de 10% en poids, une grande vitesse de dissolution dans l'eau, et forme dans l'eau une solution isotonique à une concentration d'environ 1 à environ 10% en poids/volume.

3. Procédé selon la revendication 1 ou 2, dans lequel ledit matériau porteur est un porteur soluble dans l'eau convenant à l'utilisation intraveineuse.

4. Procédé selon la revendication 3, dans lequel ledit matériau porteur est le sorbitol, le mannitol ou le xylitol, ou bien un acide aminé naturel.

5. Procédé selon la revendication 4, dans lequel ledit matériau porteur est le sorbitol.

6. Procédé selon la revendication 1, dans lequel le matériau porteur est le chlorure de sodium, le lactose, le dextrose ou le saccharose.

7. Procédé selon l'une quelconque des revendications précédentes, dans lequel on forme la solution en dissolvant ledit lipide, facultativement ledit composé biologiquement actif, et facultativement ledit adjuvant, dans un ou plusieurs solvants organiques.

8. Procédé selon la revendication 7, dans lequel le solvant organique est l'éthanol, le méthanol, le chloroforme, le dichlorométhane, l'éther diéthylique, le tétrachlorure de carbone, l'acétate d'éthyle, le dioxanne ou le cyclohexane.

9. Procédé selon la revendication 8, dans lequel le solvant est le méthanol, l'éthanol ou le chloroforme.

10. Variante du procédé tel qu'il est défini dans l'une quelconque des revendications 1 à 6, dans laquelle on forme la solution de revêtement en dissolvant le composé biologiquement actif facultatif et l'adjuvant facultatif dans ledit lipide, ledit lipide étant du type formant des liposomes à bas point de fusion.

11. Procédé selon l'une quelconque des revendications 1 à 9, dans lequel on revêt le matériau porteur en particules de ladite solution en mettant le matériau porteur en suspension dans la solution de composants liposomiques et en pulvérisant à sec le matériau porteur revêtu.

12. Procédé selon l'une quelconque des revendications précédentes, dans lequel le lipide est un phospholipide.

13. Procédé selon la revendication 12, dans lequel le phospholipide est une lécithine naturelle ou synthétique.

14. Procédé selon l'une quelconque des revendications précédentes, dans lequel ladite solution comporte un composé biologiquement actif qui est un médicament, un agent de contraste, une enzyme, une hormone, un "marqueur" ou un agent d'imagerie RMN.

15. Procédé selon l'une quelonque des revendications précédentes, dans lequel l'adjuvant est l'acide phosphatidique de l'oeuf, le phosphate de dicétyle ou la stéarylamine.

16. Procédé selon l'une quelconque des revendications 1 à 14, dans lequel l'adjuvant est un stérol.

17. Procédé selon la revendication 15, dans lequel l'adjuvant comprend également du caprolactame et/ou un stérol choisi entre le cholestérol, le phytostérol, l'ergostérol, le sitostérol, le pyroyglutamate de sitostérol, le 7-déhydrocholestérol, le lanostérol, et leurs mélanges.

18. Procédé selon l'une quelconque des revendications précédentes, dans lequel le composé biologiquement acitif est l'amphotéricine B.

19. Procédé selon la revendication 1, dans lequel le composé biologiquement actif est l'amphotéricine B, ledit matériau porteur est le sorbitol et ledit adjuvant comprend un stérol que est l'ergostérol.

20. Procédé selon la revendication 1, dans lequel le lipide est la lécithine d'oeuf, l'adjuvant est l'ergostérol, le cholestérol ou le phosphate de dicétyle, et le matériau porteur est le sorbitol.

21. Procédé selon la revendication 1, dans lequel le lipide est la lécithine d'oeuf, l'adjuvant est l'ergostérol ou le cholestérol, et le matériau porteur est le lactose.

22. Précurseur de liposomes stable qui, mélangé à de l'eau, forme une préparation liposomique, comprenant un matériau porteur en particules, ledit matériau porteur étant revêtu d'une fine pellicule constituée d'au moins un lipide formateur de liposomes, facultativement d'au moins un composé biologiquement actif, et facultativement d'au moins un adjuvant qui confère des propriétés avantageuses à la préparation de liposomes finale, le matériau porteur étant un matériau en particules soluble dans l'eau.

23. Précurseur de liposomes stable selon la revendication 22, dans lequel le matériau porteur est un matériau porteur physiologiquement acceptable fluide qui forme dans l'eau une solution isotonique à une concentration d'environ 1 à environ 10% en poids/volume.

24. Précurseur de liposomes stable selon la revendication 23, dans lequel le matériau porteur est le sorbitol.

25. Précurseur de liposomes stable selon l'une quelconque des revendications 22 à 24, dans lequel le composé biologiquement actif est l'amphotéricine B.

26. Précurseur de liposomes stable qui, mélangé à de l'eau, forme une préparation liposomique, comprenant un matériau porteur soluble dans l'eau, revêtu d'une fine pellicule constituée d'au moins un lipide formateur de liposomes, facultativement d'au moins un composé biologiquement actif, et facultativement d'au moins un adjuvant qui confère des propriétés avantageuses à la préparation de liposomes finale, préparé par le procédé tel qu'il est défini dans l'une quelconque des revendications 1 à 21.

27. Procédé de préparation d'une préparation de liposomes, qui consiste à exposer à de l'eau le précurseur de liposomes tel qu'il est défini dans l'une quelconque de revendications 22 à 26.